# EUROPEAN PATENT APPLICATION

(11) **EP 4 119 037 A1**
(43) Date of publication of application: **18.01.2023**
(21) Application number: 20923716.3
(22) Date of filing: 09.03.2020
(51) Int. Cl.: A61B 5/00

(54) **PROCESSING SELECTION PROGRAM, PROCESSING SELECTION SYSTEM, AND PROCESSING SELECTION METHOD**

(71) Applicant: FUJITSU LIMITED, Kawasaki-shi, Kanagawa 211-8588 (JP)
(72) Inventor: KOMABA, Yusuke, Kawasaki-shi, Kanagawa 211-8588 (JP); YAGINUMA, Yoshinori, Kawasaki-shi, Kanagawa 211-8588 (JP)
(74) Representative: Hoffmann Eitle
(86) International application number: PCT/JP2020/010062
(87) International publication number: WO 2021/181469

(57) **Abstract**

A processing selection program causes a computer to execute processing for acquiring sensor data measured by a sensor that measures information regarding a measurement target, processing for referring to a correspondence relationship between measurement status information and a plurality of processing programs and selecting a processing program for the sensor data according to the measurement status information of the sensor data, and processing for executing the selected processing program on the sensor data.

## Description

### FIELD

This case relates to a processing selection program, a processing selection system, and a processing selection method.

### BACKGROUND

A technique has been disclosed that collects sensor data measured by a sensor via an electric communication line or the like and makes predetermined decision (for example, refer to Patent Documents 1 to 3).

### CITATION LIST

### PATENT DOCUMENT

Patent Document 1: International Publication Pamphlet No. WO 2013/179359
Patent Document 2: International Publication Pamphlet No. WO 2015/162816
Patent Document 3: Japanese Laid-open Patent Publication No. 2007-303989

### SUMMARY

### [TECHNICAL PROBLEM]

A technique has been desired for obtaining necessary information by processing sensor data measured by a sensor using a predetermined processing program. In many cases, a developer of the processing program does not assume data other than sample data used in a verification process as input data. Therefore, processing accuracy of each processing program deteriorates for sensor data that is not assumed by the developer. Therefore, it is desirable to select an appropriate processing program for each piece of the sensor data. However, there is a case where a form of each processing program does not match a form of the sensor data assumed at the time of development of each processing program.

In one aspect, an object of this case is to provide a processing selection program, a processing selection system, and a processing selection method that can select a processing program according to sensor data.

### [SOLUTION TO PROBLEM]

In one aspect, a computer is caused to execute processing for acquiring sensor data measured by a sensor that measures information regarding a measurement target, processing for referring to a correspondence relationship between measurement status information and a plurality of processing programs and selecting a processing program for the sensor data according to the measurement status information of the sensor data, and processing for executing the selected processing program on the sensor data.

### [ADVANTAGEOUS EFFECTS OF INVENTION]

A processing program according to sensor data can be selected.

### BRIEF DESCRIPTION OF DRAWINGS

FIG. 1 are diagrams illustrating a case where a sensor is correctly attached to an ankle of a healthy person, and a case where a sensor attached to the ankle of the healthy person is not attached at a correct angle with respect to the ankle;
FIG. 2A is a diagram illustrating an entire configuration of a processing selection system, and FIG. 2B is a block diagram for explaining a hardware configuration of a processing selection device;
FIG. 3 is a flowchart illustrating an example of a flow of an operation executed by the processing selection device by selecting a processing program used as sensor data received from the sensor;
FIG. 4 is a diagram illustrating information associated with the sensor data;
FIGs. 5A to 5G are diagrams respectively illustrating history tables extracted from a correspondence relationship stored in a storage unit for each item of measurement status information;
FIG. 6A is a diagram illustrating a selection basis space used to select the processing program, and FIG. 6B is a diagram mapping a check result of a comparison unit in the selection basis space;
FIG. 7 is a diagram illustrating an adoption rate of each processing program adopted by a doctor;
FIGs. 8A to 8C are diagrams illustrating display results of a display device; and
FIGs. 9A to 9C are diagrams illustrating other examples of a processing program selection method.

### DESCRIPTION OF EMBODIMENTS

Prior to description of an embodiment, an outline of processing of sensor data will be described.

For example, in data medical care, information such as treatment and medication information, lifestyles, or genomic information is collected from various sensors, and highly personalized medical care is realized. In such data medical care, it is possible to capture symptoms that appear in daily life, which are difficult to find by a short-time hospital examination, and it is possible to realize detailed medical care. Furthermore, in a medical setting, a workload can be reduced.

Recent technological innovations have rapidly advanced in sensor precision and price reduction, and a high-accuracy sensor is more accessible to consumers. On the other hand, a form of data is becoming a barrier to processing. For example, at the beginning, measurement by a sensor under a control environment of experts such as engineers has been performed. However, measurement under a non-control environment, for example, daily life of an in-home patient is measured in industries (medical workers or the like) that is not familiar with sensor knowledge has been increased.

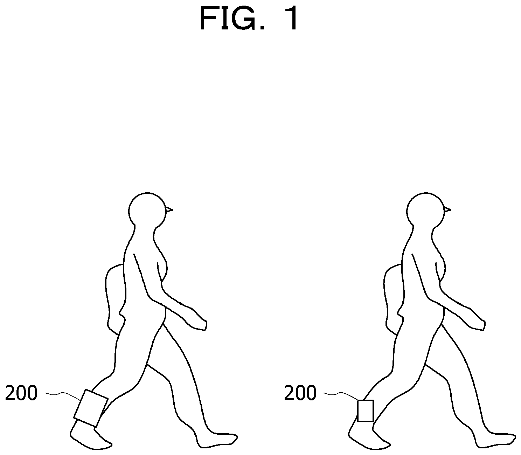

Therefore, even if measurement targets are the same, data forms (variation or bias) are different, and this affects data processing. Here, a case will be examined where sensors are attached to both ankles and sensor data at the time when a healthy person walls is processed so as to detect a walking section. FIG. 1 are diagrams illustrating a case where a sensor 200 is correctly attached to an ankle of a healthy person, and a case where a sensor 200 attached to the ankle of the healthy person is not attached at a correct angle with respect to the ankle.

In a case where the sensor 200 is not attached to the ankle at a correct angle as illustrated in FIG. 1, a processing engine (processing program) for walking feature calculation developed with an assumption of sensor data of a healthy person at the time of walking cannot detect a correct walking section, and accuracy of the calculated walking feature is deteriorated. In this case, if the processing program is used that detects that the attached sensor is not attached at the correct angle and calculates the walking feature through correction, the deterioration in the accuracy of the walking feature is suppressed. However, in a case where the sensor 200 is attached to the ankle at the correct angle as illustrated in FIG. 1, when the sensor data is processed using this processing program for correction, the accuracy of the walking feature is deteriorated than that in a case where a normal processing program is used.

In many cases, a developer of the processing program does not assume data other than sample data used in a verification process as input data. Therefore, processing accuracy of each processing program deteriorates for sensor data that is not assumed by the developer. Because it is difficult to create a versatile processing program that can cope with various cases, various processing programs are created. Therefore, in order to obtain desired processing accuracy, it is desirable to select an appropriate processing program for each piece of the sensor data.

However, there are many cases where a form of each processing program does not match a form of the sensor data assumed at the time of development of each processing program. It is difficult for one company to cope with all of this. Therefore, a platform has begun to appear that collects various processing programs of a plurality of companies and provides processing results, in order to improve processing accuracy for various forms of sensor data. However, because an operator of the platform does not understand characteristics of individual processing programs, the operator does not know which sensor data is suitable for which processing program.

Therefore, in the following embodiment, a processing selection program, a processing selection system, and a processing selection method that can select a processing program according to a form of sensor data will be described.

### [Embodiments]

In the embodiment, for example, an example will be described where a physiotherapist transmits sensor data obtained by attaching a sensor to an ankle of a patient to be measured and a processing selection system selects and executes an appropriate processing program so as to provide a walking feature to a doctor.

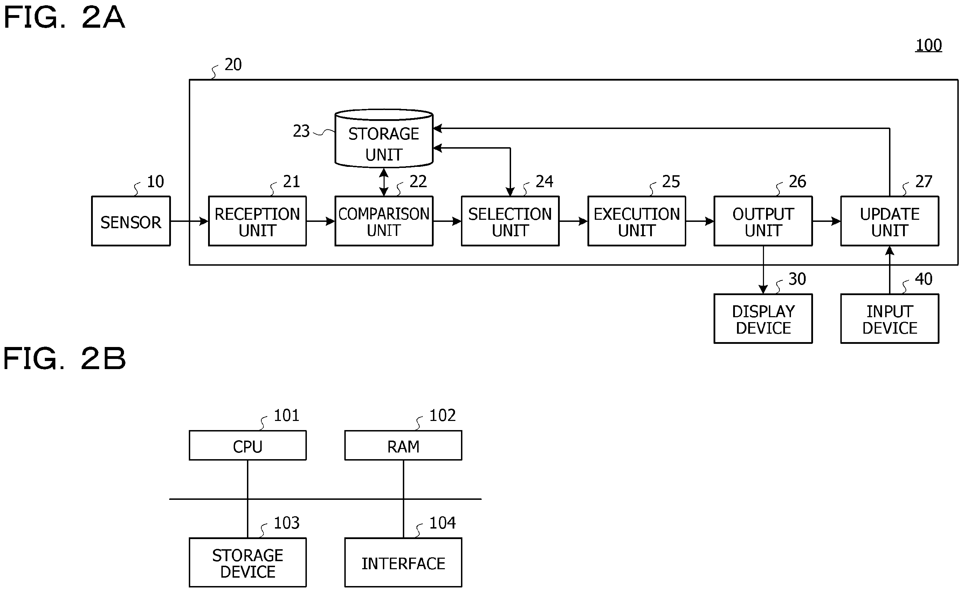

FIG. 2A is a diagram illustrating an entire configuration of a processing selection system 100. As illustrated in FIG. 2A, the processing selection system 100 includes a sensor 10, a processing selection device 20, a display device 30, an input device 40, or the like. The processing selection device 20 functions as a reception unit 21, a comparison unit 22, a storage unit 23, a selection unit 24, an execution unit 25, an output unit 26, an update unit 27, or the like.

FIG. 2B is a block diagram for explaining a hardware configuration of the processing selection device 20. As illustrated in FIG. 2B, the processing selection device 20 includes a CPU 101, a RAM 102, a storage device 103, an interface 104, or the like. These components are respectively connected to one another by a bus or the like. The central processing unit (CPU) 101 is a central processing device. The CPU 101 includes one or more cores. The random access memory (RAM) 102 is a volatile memory that temporarily stores a program to be executed by the CPU 101, data to be processed by the CPU 101, and the like. The storage device 103 is a nonvolatile storage device. For example, a read only memory (ROM), a solid state drive (SSD) such as a flash memory, a hard disk to be driven by a hard disk drive, or the like may be used as the storage device 103. By executing a processing selection program stored in the storage device 103 by the CPU 101, the reception unit 21, the comparison unit 22, the storage unit 23, the selection unit 24, the execution unit 25, the output unit 26, the update unit 27, or the like are implemented in the processing selection device 20. Note that each unit of the processing selection device 20 may be hardware such as a dedicated circuit.

The display device 30 is a device that displays a processing result of the processing selection device 20 and is a display such as a liquid crystal device. The input device 40 is an input device such as a mouse or a keyboard.

The sensor 10 is attached to a measurement target by a user of a sensor. In the present embodiment, the user of the sensor is a physiotherapist, for example. Furthermore, in the present embodiment, the measurement target is a patient to be measured such as an outpatient or an in-home patient. The sensor 10 is, for example, a gyro sensor, which detects an angular acceleration, an angular velocity, or the like. If the sensors 10 are attached to both ankles of the patient to be measured or the like, it is possible to collect data regarding walking.

The physiotherapist acquires data detected by the sensor 10 using a laptop computer or the like, and creates sensor data by cutting data in a predetermined time length range on one's own judgement. The sensor 10 has a timer function. As a result, the sensor 10 can store a measurement time period (measurement date and time or the like) when data is acquired. Note that identification information (sensor ID) is determined for the sensor 10. Furthermore, a plurality of the sensors 10 may be provided. The physiotherapist attaches own identification information (physiotherapist ID), identification information of the patient to be measured (patient to be measured ID), a patient state, an attachment time needed to attach the sensor 10, and then, transmits the sensor data via a predetermined access point. The patient state is, for example, a bedridden person, a healthy person, or the like. The predetermined access point is, for example, an access point installed in a facility where the sensor 10 performs measurement or the like.

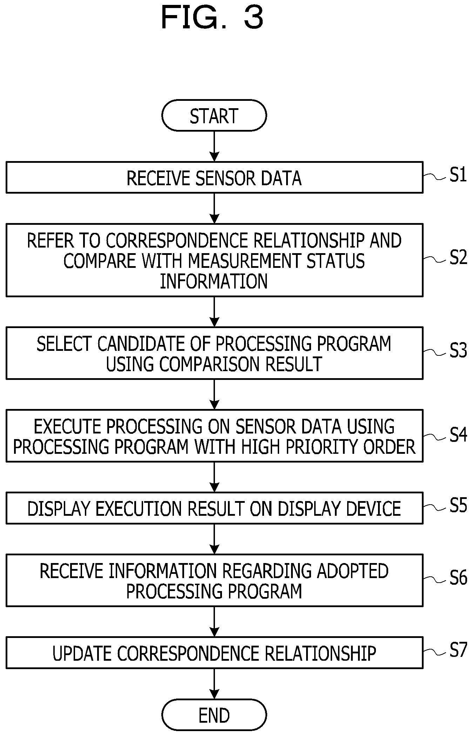

Subsequently, an operation of the processing selection device 20 will be described. FIG. 3 is a flowchart illustrating an example of a flow of an operation executed by the processing selection device 20 by selecting the processing program used as the sensor data received from the sensor 10.

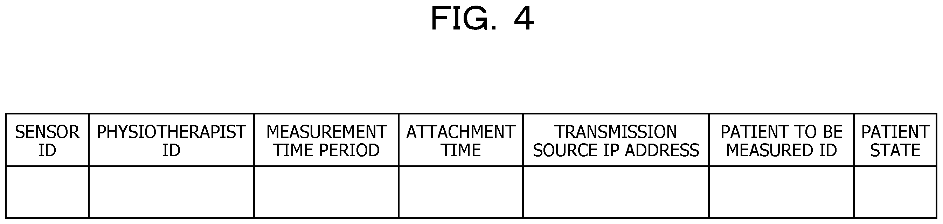

As illustrated in FIG. 3, the reception unit 21 receives the sensor data from the sensor 10 via a wired line, a wireless line, or the like (step S1). For example, as illustrated in FIG. 4, the sensor data is associated with information such as the sensor ID, the physiotherapist ID, the measurement time period, the attachment time, a transmission source IP address, the patient to be measured ID, the patient state, or the like. Furthermore, a time length of the sensor data can be used as information regarding an adopted data length adopted by the physiotherapist. From these pieces of sensor data information, measurement status information of the sensor data can be extracted. The measurement status information is information indicating a status when the sensor measures a target. In the present embodiment, the measurement status information includes a person to be measured, a measurement environment, a used device (sensor), the measurement time period, the attachment time, the patient, and the patient state. However, the measurement status information is not limited to these.

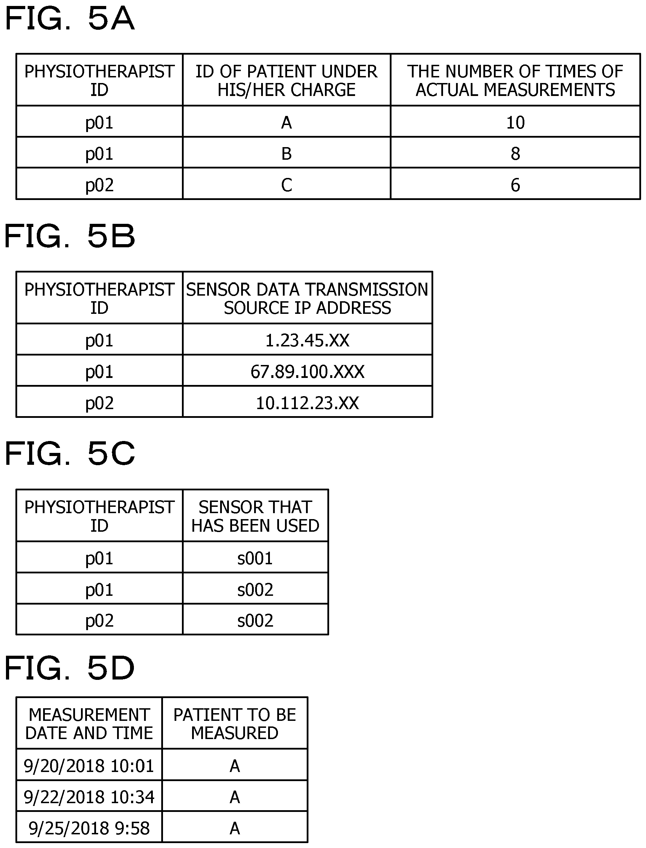

Next, the comparison unit 22 refers to the correspondence relationship stored in the storage unit 23 and compares the correspondence relationship with the measurement status information of the sensor data of this time received from the sensor 10 (step S2). The correspondence relationship stored in the storage unit 23 is a correspondence relationship between the measurement status information and the plurality of processing programs for processing the sensor data. For example, the correspondence relationship is a correspondence relationship between a processing program executed on past sensor data and measurement status information of the sensor data. FIGs. 5A to 5G are diagrams respectively illustrating history tables extracted from the correspondence relationship stored in the storage unit 23, for each item of the measurement status information. Note that the processing program is a program for executing some processing on the sensor data and is, for example, a program for extracting a feature.

FIG. 5A illustrates a history table of information regarding the user of the sensor. As illustrated in FIG. 5A, each physiotherapist ID is associated with an ID of a patient under his/her charge and the number of times of actual measurements. The patient under his/her charge is a patient to be measured who has previously acquired sensor data by attaching the sensor by the physiotherapist. For example, in the example in FIG. 5A, a physiotherapist p01 is in charge of a patient A under his/her charge and has a measurement record of 10 times for the patient A under his/her charge.

FIG. 5B illustrates a history table of information regarding a measurement environment by the user of the sensor. The measurement environment is a measurement place or the like. The measurement environment can be grasped, for example, from the transmission source IP address of the sensor data transmitted from the sensor 10 or the like. Therefore, in the example in FIG. 5B, each physiotherapist ID is associated with a transmission source IP address representing a facility where the physiotherapist has performed measurement.

FIG. 5C illustrates a history table of identification information of a sensor. In the example in FIG. 5C, each physiotherapist ID is associated with a sensor ID that has been used by the physiotherapist.

FIG. 5D illustrates a history table of a measurement time period of sensor data. As illustrated in FIG. 5D, the measurement time period is associated with the patient to be measured ID.

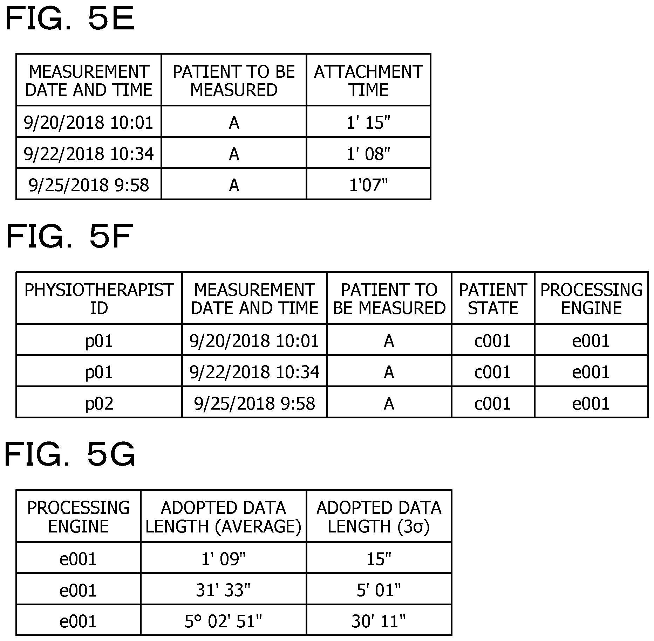

FIG. 5E illustrates a history table of an attachment time. As illustrated in FIG. 5E, the measurement time period is associated with the patient to be measured ID and the attachment time of the sensor 10.

FIG. 5F illustrates a history table of a state of a measurement target. As illustrated in FIG. 5F, each physiotherapist ID is associated with the measurement time period when the sensor 10 acquires the sensor data, the patient to be measured ID, the patient state, and the processing program used for processing of the sensor data.

FIG. 5G illustrates a history table of an adopted data length. The adopted data length is a time length of data adopted for processing among the collected sensor data, by each processing program. As illustrated in FIG. 5G, an ID of each processing program is associated with a data length adopted to calculate each statistic amount (average value, standard deviation o, or the like).

The comparison unit 22 compares the measurement status information of the sensor data received by the reception unit 21 with the correspondence relationship stored in the storage unit 23. For example, by comparing the history table in FIG. 5A, with the physiotherapist ID and the patient to be measured ID attached to the sensor data, a measurement history, the number of times of actual measurements, or the like for the patient to be measured by the physiotherapist can be determined. Furthermore, by comparing the history table in FIG. 5B with the physiotherapist ID and the transmission source IP address attached to the sensor data, it can be determined whether or not the physiotherapist has performed measurement in a facility with no measurement record, or the like. Furthermore, by comparing the history table in FIG. 5C, with the physiotherapist ID and the sensor ID attached to the sensor data, it can be determined whether or not the used sensor is a sensor that has been used by the physiotherapist in the past. Furthermore, by comparing the history table in FIG. 5D with the measurement date and time and the patient to be measured ID attached to the sensor data, a difference between the measurement time period of the sensor data for the patient to be measured and a past measurement time period, or the like can be determined. Furthermore, by comparing the history table in FIG. 5E with the patient to be measured ID and the attachment time the sensor data, a difference between the attachment time of the patient to be measured and a past attachment time or the like can be determined. Furthermore, by comparing the history table in FIG. 5F with the physiotherapist ID and the patient state attached to the sensor data, it can be determined whether or not the patient state is a patient state that has been a measurement target of the physiotherapist in the past, or the like. Furthermore, by comparing the history table in FIG. 5G with the time length of the sensor data, a difference from a data length adopted by any one of processing programs in the past or the like can be determined. The comparison results by the comparison unit 22 are transferred to the selection unit 24.

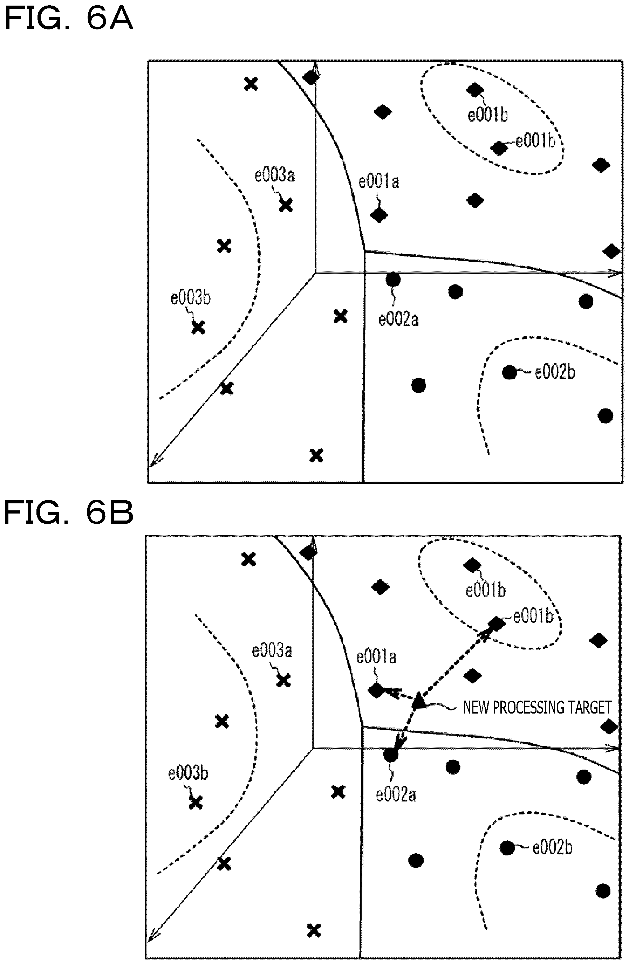

The selection unit 24 selects one or more appropriate processing programs to be executed using the comparison result by the comparison unit 22 (step S3). FIG. 6A is a diagram illustrating a selection basis space used to select a processing program. This selection basis space is the correspondence relationship stored in the storage unit 23 represented as a space. As illustrated in FIG. 6A, the selection basis space has a plurality of space axes. Each space axis represents each item of the measurement status information. In the example in FIG. 6A, the number of space axes is set to three to simplify the description. However, in the present embodiment, because the measurement status information is compared about seven measurement status items, the number of space axes is seven. For example, by mapping a relationship between the measurement status information of the sensor data collected in the past and the processing program adopted to process each piece of the sensor data in the selection basis space, a distribution of each processing program can be obtained. By partitioning the distribution of each processing program using a threshold, a coordinate range of each processing program in the selection basis space can be defined.

FIG. 6B is a diagram in which a check result of the comparison unit 22 is mapped in this selection basis space. The selection unit 24 calculates a distance between the check result and each processing program in the selection basis space. As the distance in this case, for example, the Mahalanobis distance or the like can be used. A position of each processing program can be set as a center point of the coordinate range of each processing program or the like. The selection unit 24 preferentially selects a plurality calculated distances from the smallest one as candidates. In this way, it is possible to select the processing program that has processed the sensor data measured in the past measurement status that matches the measurement status information of the sensor data of this time. Even if there is no past measurement status that matches the measurement status information of the sensor data of this time, it is possible to select the processing program that has processed sensor data measured in a close measurement status. Therefore, it is possible to select a processing program with high processing accuracy with respect to the sensor data.

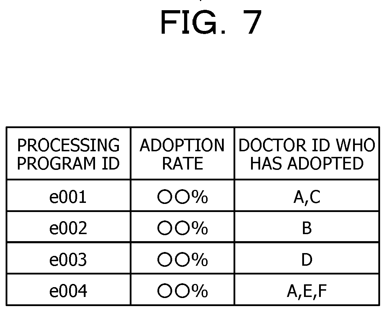

Furthermore, as illustrated in FIG. 7, an adoption rate at which each processing program is adopted by a doctor is stored in the storage unit 23 in association with each processing program. In the example in FIG. 7, each processing program ID is associated with the adoption rate and an ID of a doctor who has adopted the processing program.

Next, the execution unit 25 executes processing on the sensor data using the plurality of processing programs with high priority order from among the selected candidates (step S4). In the present embodiment, each processing program specifies a walking section in the sensor data and extracts a walking feature. Note that it is possible not to adopt a processing program of which a processing time exceeds a threshold. In this case, the execution unit 25 may execute the processing on the sensor data using the processing program with the next highest priority order among the selected candidates.

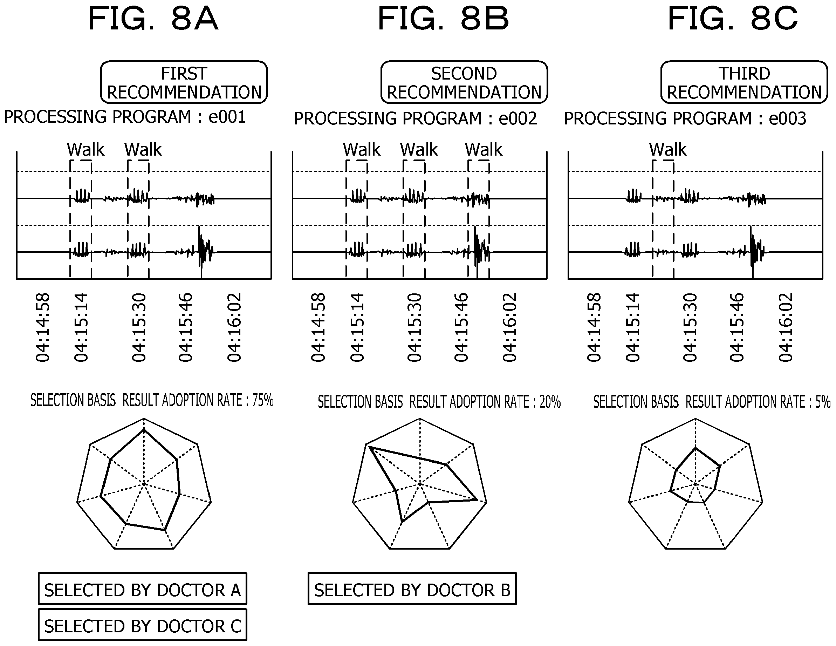

Next, the output unit 26 outputs an execution result of the execution unit 25 to the display device 30 (step S5). As a result, the display device 30 displays the execution result of the execution unit 25. FIGs. 8A to 8C are diagrams illustrating display results of the display device 30. In the examples in FIGs. 8A to 8C, the specified walking sections in the respective processing results are different. Furthermore, matching degrees between each item of the measurement status of the sensor data and the seven types of history tables stored in the storage unit 23 or the like are displayed as a heptagonal graph. For example, each processing program with the highest adoption rate at which the processing program is adopted by the doctor may be displayed with the highest recommendation order. Alternatively, each processing program having the shortest time for the processing on the sensor data of this time may be displayed with the highest recommendation order.

By referring to these display results, the doctor can determine to adopt a processing result of which processing program. For example, the doctor inputs information regarding the processing program to be adopted (processing program ID or the like) using the input device 40. As a result, the processing selection device 20 receives the information regarding the processing program adopted by the doctor (step S6). Next, the update unit 27 reflects the measurement status information of the sensor data of this time and a name of the adopted processing program on the correspondence relationship stored in the storage unit 23 so as to update the correspondence relationship (step S7). For example, the update unit 27 adds the measurement status information of the sensor data of this time to each history table. Furthermore, the update unit 27 updates the coordinate range of the processing program by mapping the name of the adopted processing program on the coordinates of the selection basis space corresponding to the measurement status of the sensor data of this time.

According to the present embodiment, by receiving the sensor data measured by the sensor that measures the information regarding the measurement target, referring to the correspondence relationship between the measurement status information and the plurality of processing programs, and selecting the processing program for the sensor data according to the measurement status information of the sensor data, it is possible to select the processing program according to the sensor data.

By displaying the execution result of the plurality of selected processing programs on the display device 30, the user can adopt a desired processing program from among the plurality of processing programs.

By updating the correspondence relationship by reflecting the measurement status information and the information regarding the processing program that has output the execution result adopted by the user on the correspondence relationship between the measurement status information and the plurality of processing programs, accuracy of the correspondence relationship is increased.

By displaying the past adoption rate of each processing program when the execution results of the plurality of selected processing programs are displayed on the display device 30, the user can consider an adoption rate of another user.

By displaying the information regarding the processing time when the execution results of the plurality of selected processing programs are displayed on the display device 30, the user can adopt a processing program that can be processed in a short time.

Note that, in the embodiment described above, since the sensor 10 is a specific sensor for walking section detection, the number of types of the sensor is one. However, there may be a plurality of types of sensors. In this case, it is sufficient to individually store a correspondence relationship between measurement status information and a processing program in the storage unit 23 for each sensor type. By referring to a metafile or the like of the sensor data, it is possible to identify which type of sensor the sensor data is. Therefore, it is possible to use a correspondence relationship that matches the sensor type among the correspondence relationship stored in the storage unit 23.

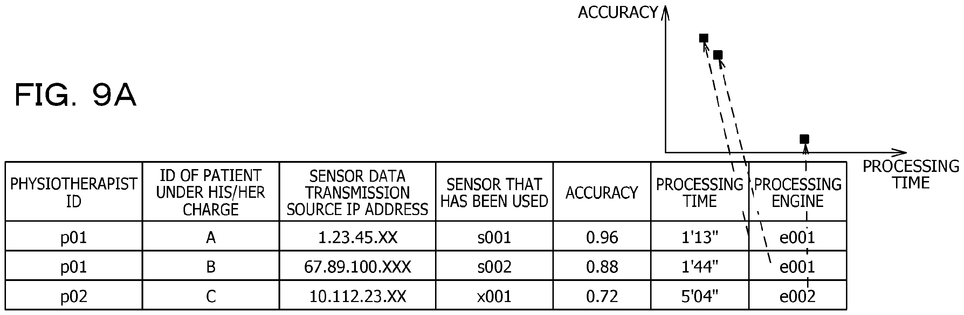

In the embodiment described above, the selection basis space is used. However, the embodiment is not limited to this. FIGs. 9A to 9C are diagrams illustrating other examples of the processing program selection method. First, as illustrated in FIG. 9A, in a preparatory process, mapping is performed on a space of accuracy of a processing result and a processing time for each non-numerical value of a measurement status profile.

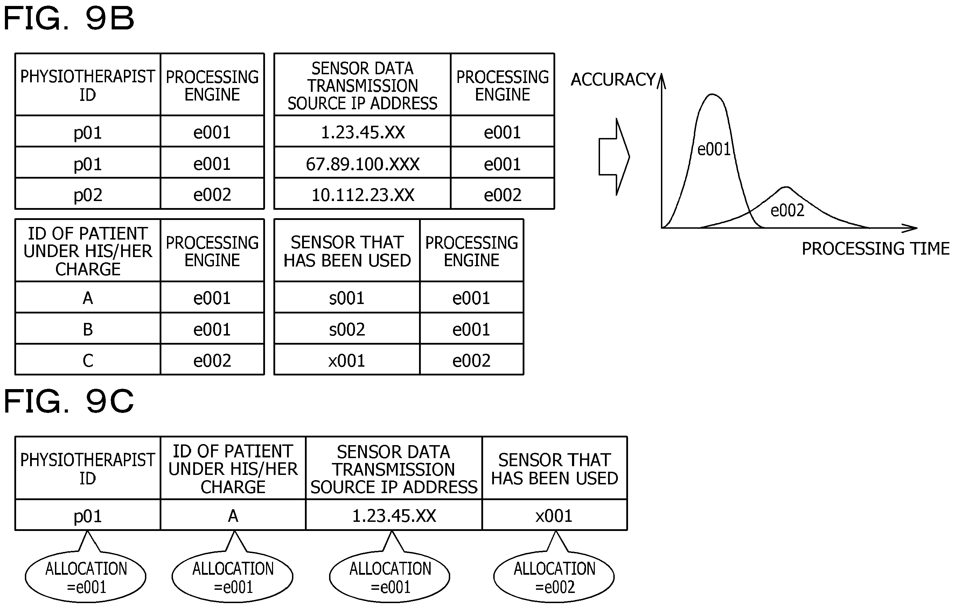

Next, as illustrated in FIG. 9B, a distribution is separated for each processing program, and a corresponding processing program of each value is allocated. For example, by using a mixture distribution model or the like, the distribution of the processing program can be separated.

In a case where new sensor data is collected, as illustrated in FIG. 9C, allocation may be searched and a processing program may be selected. A method of determination may be in an order of majority. For example, in the example in FIG. 9C, a processing program used first is e001. Referring to a processing time and accuracy that can be scaled in the processing, and if the processing time and the accuracy do not match assumption of e001, the processing program is switched to a next allocation processing program e002.

In each example described above, the sensor 10 functions as an example of a sensor that measures information regarding a measurement target. The reception unit 21 functions as an example of an acquisition unit that acquires sensor data measured by the sensor. Note that an output destination of the sensor 10 can be a storage device (not illustrated). Then, by reading data from the storage device (not illustrated), the reception unit 21 can be appropriately changed to acquire the data or the like. The selection unit 24 functions as an example of a selection unit that refers to a correspondence relationship between measurement status information and a processing program and selects a processing program for the sensor data according to the measurement status information of the sensor data. The execution unit 25 functions as an example of an execution unit that executes the selected processing program on the sensor data. The display device 30 functions as an example of a display device that displays the execution results of the plurality of selected processing programs. The input device 40 functions as an example of an input device that inputs information regarding the processing program that outputs the execution result adopted by the user, from among the execution results displayed on the display device. The update unit 27 functions as an example of an update unit that updates the correspondence relationship by reflecting the measurement status information and the information regarding the processing program that outputs the execution result adopted by the user on the correspondence relationship.

While the embodiment of the present invention has been described above in detail, the present invention is not limited to such a specific embodiment, and various modifications and alterations may be made within the scope of the present invention described in the claims.

### REFERENCE SIGNS LIST

10sensor
20processing selection device
21reception unit
22comparison unit
23storage unit
24selection unit
25execution unit
26output unit
27update unit
30display device
40input device
100processing selection system

## Claims

1. A processing selection program for causing a computer to execute processing comprising:
acquiring sensor data measured by a sensor that measures information regarding a measurement target;
referring to a correspondence relationship between measurement status information and a plurality of processing programs and selecting a processing program for the sensor data according to the measurement status information of the sensor data; and
executing the selected processing program on the sensor data.

2. The processing selection program according to claim 1, wherein the measurement status information of the sensor data includes information regarding a user of the sensor, information regarding a measurement environment of the sensor, identification information of the sensor, a measurement time period of the sensor data, an attachment time needed to attach the sensor to the measurement target, a state of the measurement target, a time length of the sensor data, or any combination thereof.

3. The processing selection program according to claim 1 or 2, wherein the number of selected processing programs is plural,
the processing selection program causes the computer to execute processing comprising:
displaying execution results of the plurality of selected processing programs on a display device; and
receiving information regarding a processing program that outputs an execution result adopted by a user from among the execution results displayed on the display device.

4. The processing selection program according to claim 3, for causing the computer to execute processing comprising:
updating the correspondence relationship by reflecting measurement status information and information regarding the processing program that outputs the execution result adopted by the user on the correspondence relationship.

5. The processing selection program according to claim 3 or 4, wherein when the execution results of the plurality of selected processing programs are displayed on the display device, past adoption rates of the plurality of selected processing programs are displayed.

6. The processing selection program according to claim 3 or 4, wherein when the execution results of the plurality of selected processing programs are displayed on the display device, information regarding a processing time needed to execute each processing program is displayed together.

7. A processing selection system comprising:
a sensor that measures information regarding a measurement target;
an aquation unit that acquires sensor data measured by the sensor;
a selection unit that:
refers to a correspondence relationship between measurement status information and a plurality of processing programs, and
selects a processing program for the sensor data according to the measurement status information of the sensor data; and
an execution unit that executes the selected processing program on the sensor data.

8. The processing selection system according to claim 7, wherein the measurement status information of the sensor data includes information regarding a user of the sensor, information regarding a measurement environment of the sensor, identification information of the sensor, a measurement time period of the sensor data, an attachment time needed to attach the sensor to the measurement target, a state of the measurement target, a time length of the sensor data, or any combination thereof.

9. The processing selection system according to claim 7 or 8, wherein the number of selected processing programs is plural,
the processing selection system further comprising:
a display device that displays execution results of the plurality of selected processing programs; and
an input device that inputs information regarding a processing program that outputs an execution result adopted by a user from among the execution results displayed on the display device.

10. The processing selection system according to claim 9, further comprising
an update device that updates the correspondence relationship by reflecting measurement status information and information regarding the processing program that outputs the execution result adopted by the user on the correspondence relationship.

11. The processing selection system according to claim 9 or 10, wherein the display device further that displays past adoption rates of the plurality of selected processing programs when displaying the execution results of the plurality of selected processing programs.

12. The processing selection system according to claim 9 or 10, wherein the display device further that displays information regarding a processing time needed to execute each processing program together when displaying the execution results of the plurality of selected processing programs.

13. A processing selection method for a computer to execute a process comprising:
acquiring sensor data measured by a sensor that measures information regarding a measurement target;
referring to a correspondence relationship between measurement status information and a plurality of processing programs and selecting a processing program for the sensor data according to the measurement status information of the sensor data; and
executing the selected processing program on the sensor data.

14. The processing selection method according to claim 13, wherein the measurement status information of the sensor data includes information regarding a user of the sensor, information regarding a measurement environment of the sensor, identification information of the sensor, a measurement time period of the sensor data, an attachment time needed to attach the sensor to the measurement target, a state of the measurement target, a time length of the sensor data, or any combination thereof.

15. The processing selection method according to claim 13 or 14,
wherein
the number of selected processing programs is plural,
the processing selection method causes the computer to execute processing comprising:
displaying execution results of the plurality of selected processing programs on a display device; and
receiving information regarding a processing program that outputs an execution result adopted by a user from among the execution results displayed on the display device.

16. The processing selection method according to claim 15, for causing the computer to execute processing comprising:
updating the correspondence relationship by reflecting measurement status information and information regarding the processing program that outputs the execution result adopted by the user on the correspondence relationship.

17. The processing selection method according to claim 15 or 16, wherein when the execution results of the plurality of selected processing programs are displayed on the display device, past adoption rates of the plurality of selected processing programs are displayed.

18. The processing selection method according to claim 15 or 16, wherein when the execution results of the plurality of selected processing programs are displayed on the display device, information regarding a processing time needed to execute each processing program is displayed together.
